# EUROPEAN PATENT APPLICATION

(11) **EP 2 953 087 A1**
(43) Date of publication of application: **09.12.2015**
(21) Application number: 13873413.2
(22) Date of filing: 29.01.2013
(51) Int. Cl.: G06Q 50/22

(54) **MEDICAL INFORMATION MANAGEMENT DEVICE, MEDICAL INFORMATION MANAGEMENT SYSTEM, AND CONTROL METHOD FOR MEDICAL INFORMATION MANAGEMENT DEVICE**

(71) Applicant: Terumo Kabushiki Kaisha, Tokyo 151-0072 (JP)
(72) Inventor: TOUNOOKA Yuuya, Ashigarakami-gun Kanagawa 259-0151 (JP); YAMADA Kazuhiro, Ashigarakami-gun Kanagawa 259-0151 (JP)
(74) Representative: Casalonga
(86) International application number: PCT/JP2013/051898
(87) International publication number: WO 2014/118885

(57) **Abstract**

[Problem] Provided is a medical information management apparatus or the like which automatically allows exceptional access to medical information under certain restriction even though a user such as hospital clerical staff or the like is restricted from having access to the medical information.

[Means for Resolution] According to a medical information management apparatus 10, when first medical information including second medical information requested for access based on user identification information is judged to be inaccessible based on user access associated information 21, degree identification information of the second medical information is specified based on third medical information 23, and the specified degree identification information and the degree identification information of the user access associated information are compared to each other so as to judge whether or not access to the second medical information is possible.

## Description

### Technical Field

The present invention relates to a medical information management apparatus for managing medical information such as drug information or the like which is used in hospitals or the like, for example, a medical information management system, and a method of controlling a medical information management apparatus.

### Background Art

Conventionally in hospitals or the like, an infusion pump or the like has been adopted in order to appropriately administration a drug to a patient (for example, PTL 1).

In regard to drug information or the like used in medical instruments or the like such as the infusion pump or the like, a database such as "a drug library" or the like is configured in order to manage information thereof, and a system is adopted in which a drug or the like can be appropriately prescribed by using the infusion pump or the like.

In hospitals, in addition to the drug information, information related to medical affairs, administrative affairs, and the like of a hospital is included in the database of the hospital, thereby being systematized so as to allow clerical staff of the hospital to easily acquire various items of overall information of the hospital from a terminal of oneself.

For this reason, "the drug library", hospital affairs management data or the like such as "the medical affairs", and the like are networked in one system. Therefore, even clerical staff in charge of the medical affairs management or the like can have access to "the drug library", and the access may cause an erroneous change of the data or the like.

Thus, it is systematized to previously designate an accessible region of data or the like for clerical staff of the hospital, thereby prohibiting the clerical staff in principle from having access to a database or the like which is irrelevant to duties.

### Citation List

### Patent Literature

PTL 1: JP-A-2011-87678

### Summary of Invention

### Technical Problem

However, for example, there is a case where even clerical staff in charge of medical affairs management or the like needs to have access to a database such as "a drug library" or the like which is rarely relevant to duties in principle. In this case, in order to have access, the clerical staff needs to acquire access authorization from an administrator of a system each time, thereby resulting in a problem that causes inconvenience in the conduction of duties.

Meanwhile, due to the characteristics of medical affairs, it is difficult to permit unrestricted access thereto. Therefore, a procedure has been required to have access to "the drug library" or the like after passing through a process of permission or the like from the administrator or the like.

Thus, the present invention aims to provide a medical information management apparatus which automatically allows exceptional access to medical information under certain restriction in order to enhance convenience for a user of the medical information even though the user such as hospital clerical staff or the like is restricted from having access to the medical information. The present invention also aims to provide a medical information management system and a method of controlling a medical information management apparatus.

### Solution to Problem

According to the present invention, the aforementioned objective is achieved by providing a medical information management apparatus which includes user identification information for identifying a user who uses medical information, a plurality of items of first medical information that are obtained by segmenting the medical information into a plurality of types, a plurality of items of second medical information that are obtained by segmenting the first medical information further into a plurality of types, a third medical information storage unit that stores third medical information for causing the second medical information to be associated with degree identification information which indicates a degree of the second medical information, and a user access associated information storage unit that stores the user identification information and user access associated information in which at least the user identification information is registered by causing the accessible first medical information to be associated with the degree identification information correlated to the user identification information. When the first medical information including the second medical information requested for access based on the user identification information is judged to be inaccessible based on the user access associated information, the degree identification information of the second medical information is specified based on the third medical information, and the specified degree identification information and the degree identification information of the user access associated information are compared to each other so as to judge whether or not access to the second medical information is possible.

According to the above-described configuration, the present invention exhibits an effect or the like when the first medical information including the second medical information requested for access based on the user identification information is judged to be inaccessible based on the user access associated information. For example, hospital clerical staff having the user identification information (for example, an ID number) is generally in charge of medical affairs, and the hospital clerical staff sometimes needs to have access to "the second medical information" such as "composing/editing" or the like of "the first medical information" such as "a drug library" or the like which is generally irrelevant to duties.

In this case, according to the above-described configuration, for example, since the hospital clerical staff having the user associated information is generally irrelevant to "the first medical information", that is, "the drug library" in the conduction of duties, the hospital clerical staff cannot have access to "the drug library". Accordingly, a possibility of a change or the like erroneously made by the hospital clerical staff in a database such as "the drug library" or the like which is irrelevant to one's duties is eliminated, thereby ensuring security in management of the medical information which a system handles.

Nevertheless, even the hospital clerical staff sometimes needs to have access to the database such as "the drug library" or the like which is generally irrelevant to one's duties. In this case, in the related art, access has been performed after completing a process causing the user associated information of the hospital clerical staff to be associated with authorization of exceptional access to the database. Thus, there is a concern of delays in the conduction of duties.

Therefore, according to the above-described configuration, the degree identification information of the second medical information is specified based on the third medical information, and the specified degree identification information and the degree identification information of the user access associated information are compared to each other so as to judge whether or not access to the second medical information is possible.

Here, the third medical information is information causing the second medical information to associate with the degree identification information which indicates degree of the second medical information.

In other words, "the second medical information" such as "composing/editing" or the like is associated with "an authorization level 3" which is the degree identification information, for example.

The degree identification information (for example, an authorization level 6 or the like) correlated to the user identification information is registered in the user access associated information storage unit by being associated therewith.

Therefore, the specified degree identification information of "the authorization level 3" and the degree identification information (for example, the authorization level 6) of the user access associated information are compared to each other, thereby determining whether or not access thereto is possible.

For example, if the degree identification information (for example, the authorization level 6) of the user access associated information is rated above the specified degree identification information of "the authorization level 3", the hospital clerical staff is automatically allowed to have access thereto.

Accordingly, certain hospital clerical staff can have temporary access to information, for example, even though the information is "the second medical information" such as "composing/editing" or the like of "the first medical information" such as "the drug library" or the like to which the hospital clerical staff generally has no access so that duties are smoothly conducted, thereby improving convenience of a user.

The access is not unconditional. The access is judged by comparing "the degree identification information" of "the second medical information" to which the hospital clerical staff intends to have access and "the degree identification information" of the user identification information of the hospital clerical staff or the like.

Therefore, it is possible to ensure security in management of the medical information and to improve convenience of a user.

Preferably, the degree identification information of the second medical information includes rating degree information which is a common criterion for each item of the first medical information. When the rating degree information of the specified degree identification information is not rated over the rating degree information of the degree identification information of the user access associated information, access requested based on the user identification information is approved on condition of temporal limitation.

According to the above-described configuration, the degree identification information included in the first medical information has items of the rating degree information, for example, an authorization level 1, an authorization level 2, the authorization level 3, and the like which are the common criterion for each item of the first medical information.

When the rating degree information of the specified degree identification information, for example, the authorization level 2 is not rated over the rating degree information (for example, the authorization level 6) of the degree identification information of the user access associated information, access requested based on the user identification information is approved on condition of temporal limitation (for example, for this once).

Therefore, even in a case of permitting access to "the second medical information" such as "composing/editing of the drug library" or the like which is different from the routine duties (previously assigned duties) of a user such as hospital clerical staff or the like, the access is limited time-wise. Thus, it is possible to ensure security in management of the medical information.

Preferably, the medical information management apparatus further includes an access history information storage unit that stores history information of access permission approved on condition of the temporal limitation. When the history information satisfies a prejudged requirement, the access is permitted without comparing the specified degree identification information and the degree identification information of the user access associated information.

According to the above-described configuration, the access history information storage unit that stores the history information of access permission approved on condition of the temporal limitation is included, and when the history information satisfies a prejudged requirement, the access is permitted without comparing the specified degree identification information and the degree identification information of the user access associated information.

In other words, when the frequency of permission of access to "the second medical information" such as "composing/editing of the drug library" or the like which is different from the routine duties (previously assigned duties) of a user such as hospital clerical staff or the like is equal to or more than a certain frequency within a certain term, for example, prompt access is assured without permitting access each time. Thus, it is possible to further smoothly conduct duties.

For example, if a prejudged requirement, that is, a requirement of being equal to or more than a certain frequency within a certain term is satisfied, there is no remarkable problem from a viewpoint of security in management of the medical information in most of cases. Therefore, it is necessary to prioritize smoothness in the conduction of duties.

Preferably, when the permitted access is stored in an exceptional access information storage unit as exceptional access information without comparing the specified degree identification information and the degree identification information of the user access associated information, if the history information satisfies a prejudged condition, the exceptional access information is cancelled.

According to the above-described configuration, when the permitted access is stored in the exceptional access information storage unit as the exceptional access information without comparing the specified degree identification information and the degree identification information of the user access associated information, if the history information satisfies a prejudged condition, the exceptional access information is cancelled.

In other words, when the frequency of permission of access to "the second medical information" such as "composing/editing of the drug library" or the like which is different from the routine duties (previously assigned duties) of a user such as hospital clerical staff or the like is equal to or more than a certain frequency within a certain term, for example, a prejudged registration is performed so as not to permit access each time. However, afterwards, if the frequency of access or the like decreases within a certain term, for example, the situation changes to ensure security in management of the medical information rather than necessity in requirement of the exceptions. Thus, the registration thereof is cancelled so as to ensure security in management of the medical information.

Preferably, a terminal device that is used by a user is included. The medical information management apparatus is communicably connected to the terminal device.

According to the present invention, the aforementioned objective is achieved by providing a method of controlling a medical information management apparatus which includes user identification information for identifying a user who uses medical information, a plurality of items of first medical information that are obtained by segmenting the medical information into a plurality of types, a plurality of items of second medical information that are obtained by segmenting the first medical information further into a plurality of types, a third medical information storage unit that stores third medical information for causing the second medical information to be associated with degree identification information which indicates a degree of the second medical information, and a user access associated information storage unit that stores the user identification information and user access associated information in which at least the user identification information is registered by causing the accessible first medical information to be associated with the degree identification information correlated to the user identification information. When the first medical information including the second medical information requested for access based on the user identification information is judged to be inaccessible based on the user access associated information, the degree identification information of the second medical information is specified based on the third medical information, and the specified degree identification information and the degree identification information of the user access associated information are compared to each other so as to judge whether or not access to the second medical information is possible.

### Advantageous Effects of Invention

As described above, according to the present invention, it is possible to provide a medical information management apparatus which automatically allows exceptional access to medical information under certain restriction in order to enhance convenience for a user of the medical information even though the user such as hospital clerical staff or the like is restricted from having access to the medical information. It is also possible to provide a medical information management system and a method of controlling a medical information management apparatus.

### Brief Description of Drawings

[Fig. 1] Fig. 1 is a schematic diagram showing "a medical information management system" of the present invention.
[Fig. 2] Fig. 2(a) is a schematic diagram showing a database of "a drug library", and Fig. 2(b) is a schematic diagram showing a database of "hospital affairs management data".
[Fig. 3] Fig. 3 is a schematic block diagram showing a main configuration of a hospital management server and a terminal for hospital clerical staff shown in Fig. 1.
[Fig. 4] Fig. 4 is a schematic block diagram showing contents of "a various-data item storage unit" shown in Fig. 3.
[Fig. 5] Fig. 5 is a schematic block showing contents of "a various-data item processing unit (program) (1)".
[Fig. 6] Fig. 6 is a schematic block showing contents of "a various-data item processing unit (program) (2)".
[Fig. 7] Fig. 7 is a schematic flowchart showing an operational example or the like of the medical information management system in an embodiment of the present invention.
[Fig. 8] Fig. 8 is another schematic flowchart showing an operational example or the like of the medical information management system in the embodiment of the present invention.
[Fig. 9] Fig. 9 is still another schematic flowchart showing an operational example or the like of the medical information management system in the embodiment of the present invention.
[Fig. 10] Fig. 10 is still another schematic flowchart showing an operational example or the like of the medical information management system in the embodiment of the present invention.

### Description of Embodiments

Hereinafter, a preferable embodiment of the invention will be described in detail with reference to the accompanying drawings or the like.

Note that, the below-described embodiment is a preferable specific example of the present invention and is applied with various types of limitation which are technically preferable. However, the scope of the present invention is not limited to the aspects thereof unless otherwise disclosed particularly limiting the present invention in the following descriptions.

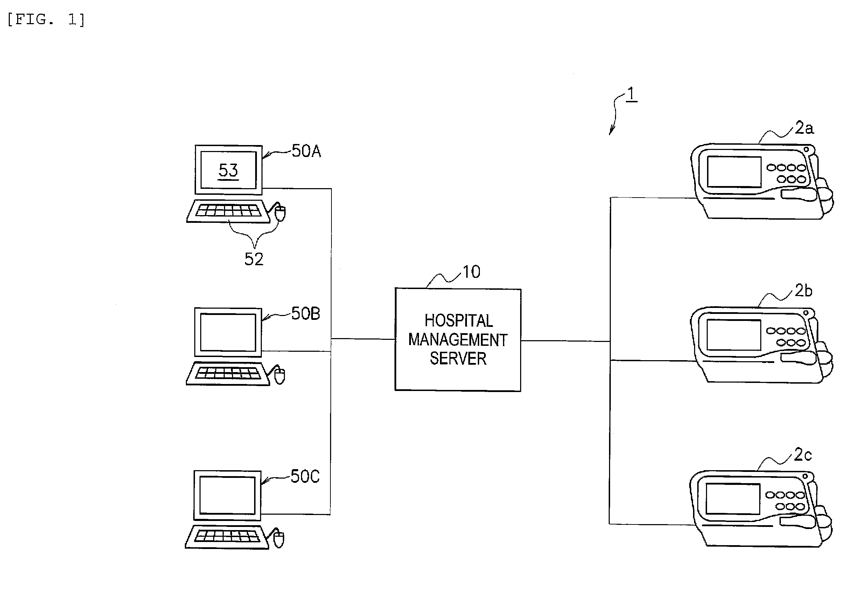

Fig. 1 is a schematic diagram showing "a medical information management system 1" of the present invention.

As shown in Fig. 1, the medical information management system 1 has a hospital management server 10 which is a medical information management apparatus, for example. or example, terminals for hospital clerical staff 50A, 50B, and 50C which are terminal devices are communicably connected to a hospital management server 1 and are communicably connected to the hospital management server 10 of infusion pumps 2a, 2b, and 2c, for example, which are medical instruments arranged in a hospital.

A database or the like of various items of data used in a hospital is stored in the hospital management server 10. he database is an example of "first medical information".

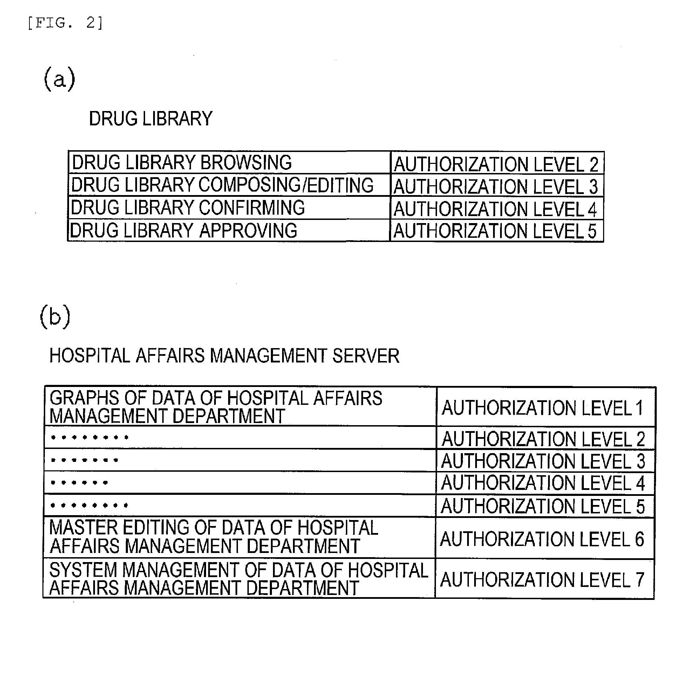

Fig. 2 is a schematic diagram showing a specific example of such a database.

Fig. 2 (a) is the schematic diagram showing the database of "a drug library".
"The drug library" includes detailed data related to prescriptions or the like of drugs (for example, types of drugs (anticancer drugs, anesthetics), drug names, upper and lower limit values of flow rates (mL/h), upper and lower limit values of injection rates (mL), and contraindication information). The data is mainly transmitted to an infusion pump 2a or the like in Fig. 1, and the data is utilized when a health care worker such as a nurse or the like operating the infusion pump 2a or the like administrations a drug to a patient.

Note that, the infusion pump 2a or the like is a medical instrument which is adopted when accurately administrating a drug to a patient, for example. The infusion pump 2a or the like is arranged in an intravenous drip device or the like if a highly accurate control is required when administrating a drug, for example.

Fig. 2 (b) is a schematic diagram showing a database of "hospital affairs management data".

"The hospital affairs management data" is data for managing medical affairs of a hospital, administrative data, and the like.

Therefore, a hospital clerical staff, that is, "The hospital affairs management data" is configured that a health care worker such as a nurse or the like, a clerical staff in charge of hospital medical affairs, a hospital executive, or the like operates "the terminal 50A for hospital clerical staff" or the like in Fig. 1, thereby being able to smoothly have access to data related to one's duties.

However, if all of the hospital clerical staff members, for example, clerical staff in charge of medical affairs are allowed to freely have access to the database of "the drug library", the data is changed due to an erroneous operation or the like, for example, resulting in a possibility of an occurrence of an unexpected accident when using "the drug library".

Therefore, access authorization is designated in the database stored in the hospital management server 10 in Fig. 1 depending on the types thereof so as to limit hospital clerical staff or the like who is irrelevant to the database from having access to the database.

For example, only health care workers or the like such as doctors, nurses, pharmacists, and the like are granted the access authorization for "the drug library" in Fig. 2 (a), and other clerical staff members in charge of the medical affairs do not qualify for the access authorization.

Note that, Fig. 2 (a) shows four levels of authorization levels 2 to 5. However, the authorization levels are not limited thereto.

Meanwhile, only the clerical staff in charge of the medical affairs and the executive staff are granted the access authorization for the database of "the hospital affairs management data" in Fig. 2(b), and the health care workers or the like such as doctors or the like are not granted the access authorization.

"The drug library", "hospital information management data", or the like is designated with a plurality of processing contents in a case of having access thereto. or example, in a case of "the drug library", processing of browsing, composing/editing, confirming, editing, or the like shown in Fig. 2 (a) is designated, and in a case of "the hospital information management data", processing of graphs, master editing, system management, or the like shown in Fig. 2(b) is designated. he processing contents are examples of "second medical information".

Then, the processing contents are designated with "the authorization level", for example, "degree identification information" executable in accordance with a degree thereof. hen, only hospital clerical staff having such "an authorization level" can execute the processing. Specifically, as described below, the authorization level is linked to an ID number, for example, "user identification information" of hospital clerical staff.

Note that, Fig. 2(b) shows seven levels of the authorization levels 1 to 7. However, the authorization levels are not limited thereto.

Incidentally, the hospital management server 10, the terminal 50A for hospital clerical staff or the like, the infusion pump 2a or the like shown in Fig. 1 has a computer. The computer has a central processing unit (CPU), a random access memory (RAM), a read only memory (ROM), and the like (not illustrated), which are connected to each other through a bus.

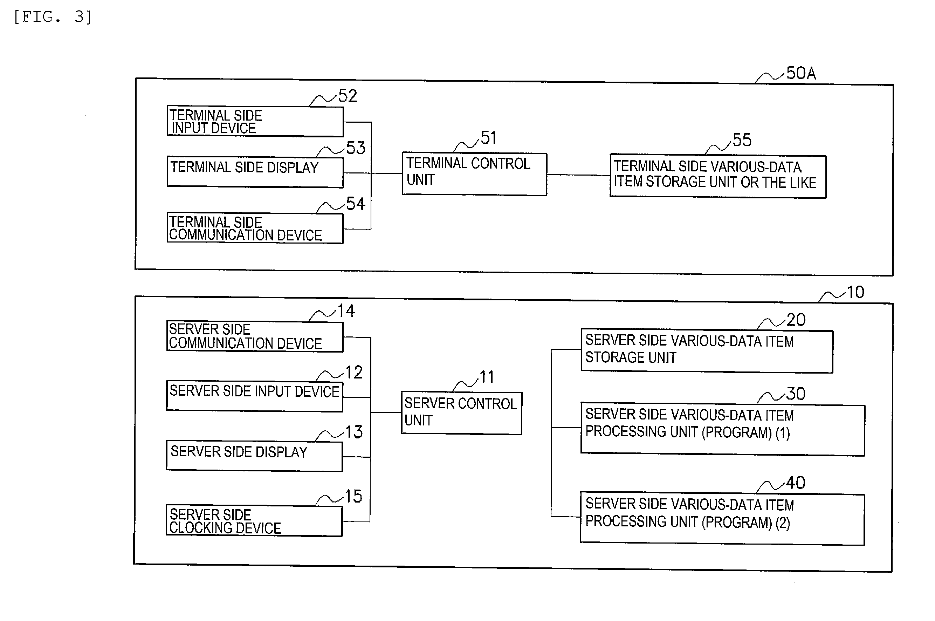

Fig. 3 is a schematic block diagram showing a main configuration of the hospital management server 10, the terminal 50A for hospital clerical staff, or the like shown in Fig. 1.

As shown in Fig. 3, the terminal 50A for hospital clerical staff or the like has a terminal control unit 51. The terminal control unit 51 is configured to controls "a terminal side input device 52" for inputting data or the like, "a terminal side display 53" for displaying data or the like, and "a terminal side communication device 54" for communicating with the hospital management server 10 or the like. The terminal control unit 51 is also configured to controls "a terminal side various-data item storage unit or the like 55".

As shown in Fig. 3, the hospital management server 10 has a server control unit 11. The server control unit 11 controls is configured to "a terminal side input device 12" for inputting data or the like, "a server side display 13" for displaying data or the like, "a server side communication device 14" for communicating with the terminal 50 for hospital clerical staff or the like, and "a server side clocking device 15". The server control unit 11 is also configured to controls "a server side various-data item storage unit 20", "a server side various-data item processing unit (program) (1) 30", and "a server side various-data item processing unit (program) (2) 40".

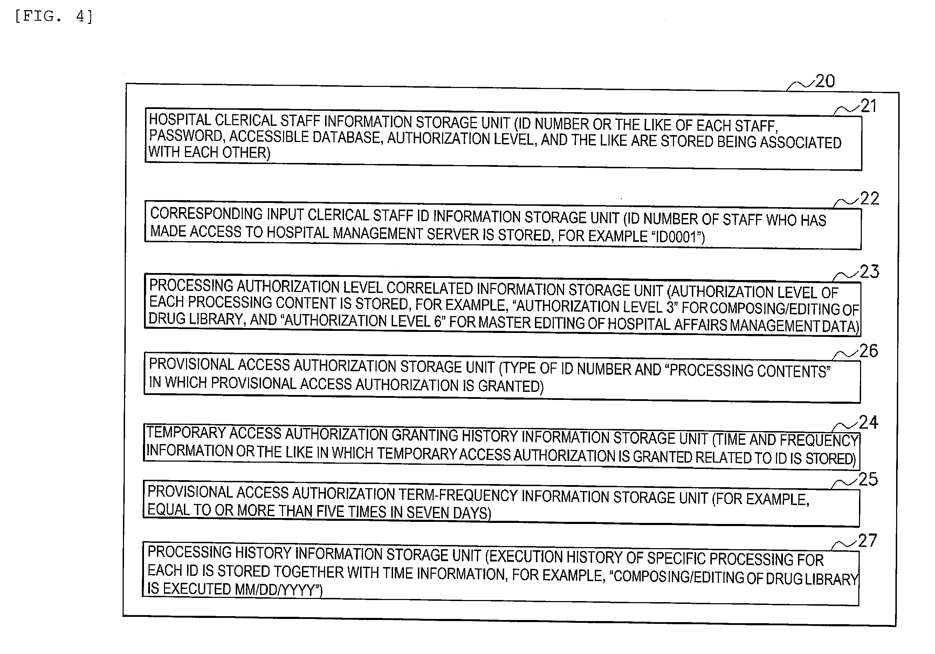

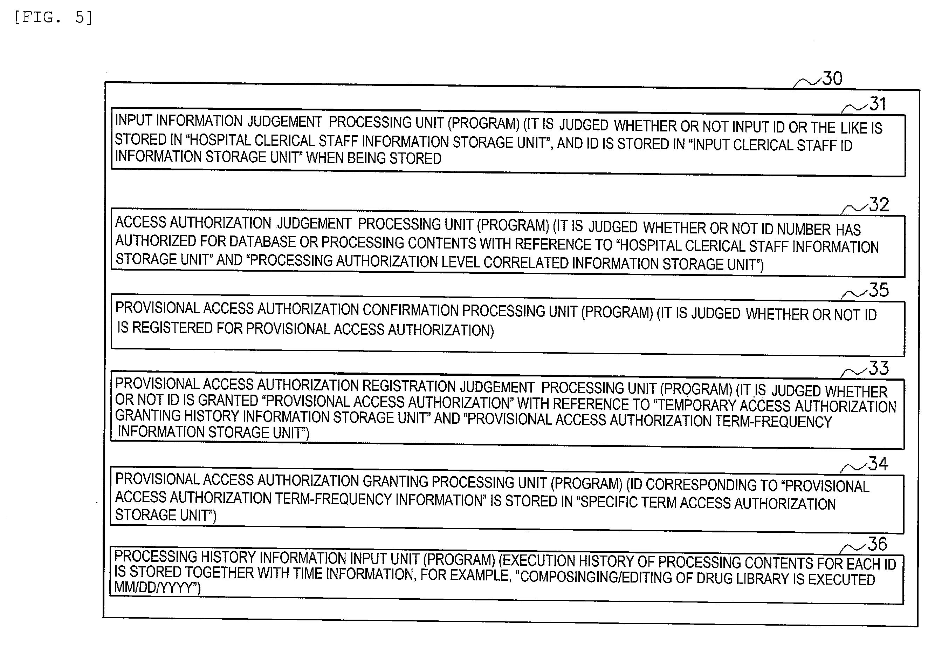

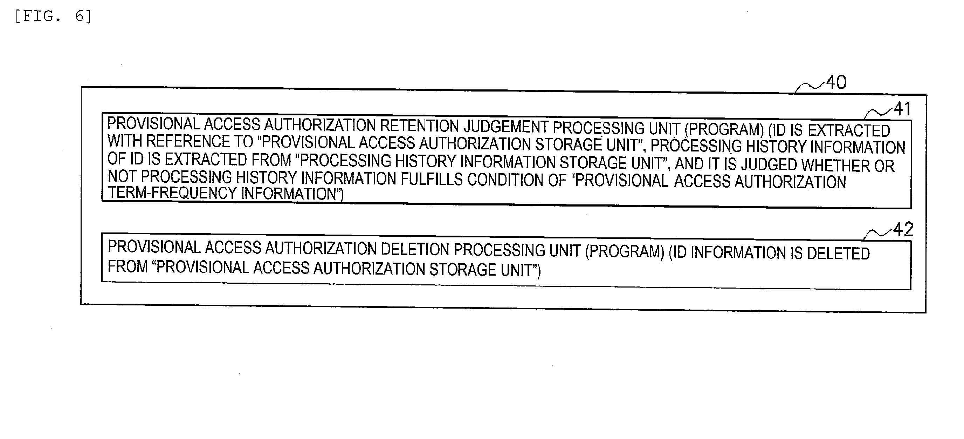

Note that, Fig. 4 is a schematic block diagram showing contents of "a various-data item storage unit 20" shown in Fig. 3. Fig. 5 is a schematic block showing contents of "the various-data item processing unit (program) (1) 30". Fig. 6 is a schematic block showing contents of "the various-data item processing unit (program) (2) 40". Each of the contents of the blocks will be described later.

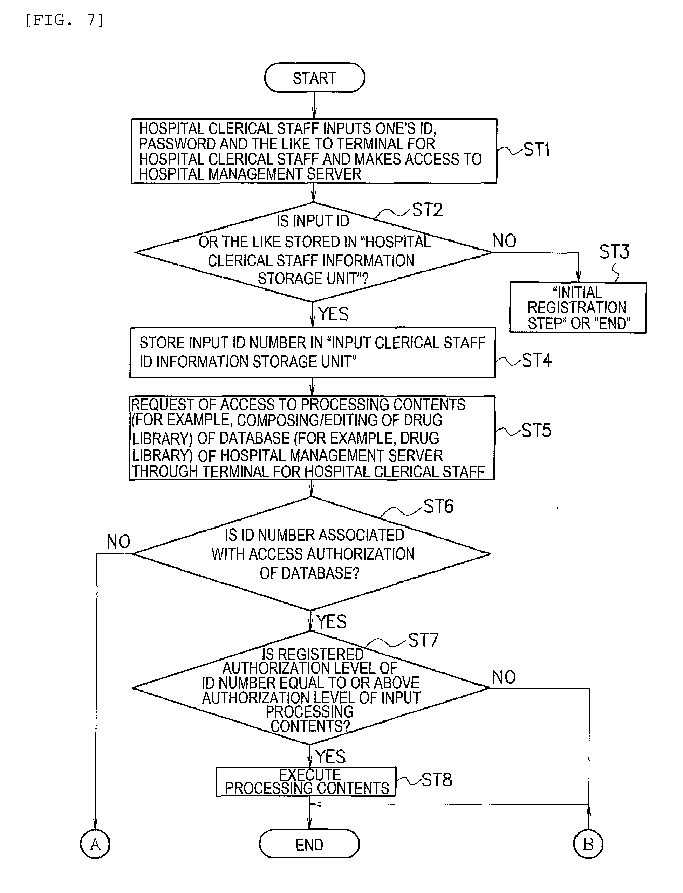

Figs. 7 to 10 are schematic flowcharts showing operational examples or the like of the medical information management system 1 in the embodiment of the present invention. Hereinafter, operations thereof will be described with reference to the flowcharts in Figs. 7 to 10, and configurations or the like in Figs. 1 to 6 will be also described.

Firstly, hospital clerical staff, for example, who is a user intending to have access to the hospital management server 10 in Fig. 1 individually has an ID number or the like, for example, the user identification information.

Therefore, the hospital clerical staff intending to have access to the hospital management server 10 in Fig. 1 uses the terminal 50A for hospital clerical staff shown in Fig. 1, and as shown in ST 1, the hospital clerical staff inputs one's ID number, a password, and the like to one's terminal 50A for hospital clerical staff.

Subsequently, in ST 2, it is judged whether or not the input ID number and the like are stored in "a hospital clerical staff information storage unit 21". As shown in Fig. 4, the ID number of hospital clerical staff and "the password" correlated to the ID number are registered in "the hospital clerical staff information storage unit 21".

Therefore, it is possible to judge whether or not an inputting person is already registered by determining whether or not the person is stored in "the hospital clerical staff information storage unit 21", and thus, it is possible to prevent an unregistered person from having access to "the hospital management server 10".

Concretely, the operation is executed by "an input information judgment processing unit (program) 31" in Fig. 5.

In ST 2, when it is judged that the input ID number and the like are not registered in "the hospital clerical staff information storage unit 21", as shown in ST 3, "an initial registration step" is executed, and initial registration is performed, or the step ends without performing the initial registration.

In ST 2, when it is judged that the input ID number and the like are registered in "the hospital clerical staff information storage unit 21", the procedure proceeds to ST 4. In ST 4, the input ID number is stored in "a corresponding input clerical staff ID information storage unit 22" in Fig. 4.

Subsequently, the procedure proceeds to ST 5.

In ST 5, hospital clerical staff inputs "the database" to which the one intends to have access through one's terminal 1A for hospital clerical staff and also inputs "the processing contents".

For example, "the drug library" is input as the database, and "composing/editing of the drug library" (refer to Fig. 2(a)) is input as the processing contents.

Subsequently, the procedure proceeds to ST 6.

In ST 6, it is judged whether or not the ID number is associated with the access authorization for the database (for example, "the drug library").

Specifically, "an access authorization judgment processing unit (program) 32" in Fig. 5 operates, thereby determining whether or not the input ID number coincides with "an accessible database" of "the hospital clerical staff information storage unit 21" in Fig. 4.

In ST 6, if the inputable database coincides with the accessible database, the procedure proceeds to ST 7.

Meanwhile, a case where the inputable database does not coincide with the accessible database in ST 7 will be described later.

In ST 7, it is judged whether or not the registered authorization level of the ID number, that is, "the authorization level" registered in "the hospital clerical staff information storage unit 21" in Fig. 4 being associated with the ID number is equal to or above "the authorization level" of "composing/editing of the drug library" which is the input processing contents.

Specifically, "the access authorization judgment processing unit (program) 32" performs the judgment thereof. In other words, the judgment is performed with reference to "a processing authorization level correlated information storage unit 23" in Fig. 4. However, "the processing authorization level correlated information storage unit 23" stores each of the processing contents, for example, "an authorization level 3" which is rating degree information of "the authorization level" of "composing/editing of the drug library" in Fig. 2 (a). "The processing authorization level correlated information storage unit 23" also stores that "master editing of the hospital affairs management data" which is the processing contents in Fig. 2(b) is "an authorization level 6", for example.

For this reason, after acquiring "the authorization level" registered in "the hospital clerical staff information storage unit 21" being associated with the ID number, "the processing authorization level correlated information storage unit 23" judges whether or not "the authorization level" is equal to or above "the authorization level 3" for the processing contents of "composing/editing of the drug library" which is stored in "the processing authorization level correlated information storage unit 23".

Therefore, "a hospital clerical staff information recording unit 21" is an example of "a user access associated information storage unit", and "the processing authorization level correlated information storage unit 23" is an example of "a third medical information storage unit".

In ST 7, if "the authorization level" associated with the ID number is not equal to or above "the authorization level 3", for example, of "composing/editing of the drug library", for example, the input processing contents, the hospital clerical staff who has input the ID number is considered not to be authorized to execute the processing contents, thereby being rejected access thereto.

Meanwhile, in ST 7, when "the authorization level" associated with the ID number is equal to or above "the authorization level 3", for example, of "composing/editing of the drug library", for example, the input processing contents, the procedure proceeds to ST 8. In ST 8, the hospital clerical staff who has input the corresponding ID number is considered to be authorized to execute "composing/editing of the drug library" which is the processing contents, thereby executing "composing/editing of the drug library" which is the processing contents.

As described above, in the present embodiment, if the hospital clerical staff is in charge of medical affairs management, for example, and is not a health care worker such as a doctor, a nurse, a pharmacist or the like, the hospital clerical staff is considered in principle not to be qualified for the access authorization for "the drug library" which is the database related to prescriptions or the like of drugs, thereby ensuring security in management of the data.

In other words, the staff in charge of the medical affairs management can have access to "the hospital affairs management data" which is the database in Fig. 2(b), thereby ensuring convenience thereof.

However, even though staff is in charge of the medical affairs management, the staff may be also in charge of administrative affairs as well as being a doctor, for example. In a case of such staff, there is a need to have access to "the drug library", for example, the database irrelevant to the routine duties, in the conduction of duties.

Even in such a case, if access to the database is rejected in the same manner and registration of the access authorization is required each time, inconvenience is caused in the conduction of duties. Thus, in the present embodiment, processing is performed as described below.

Firstly, in ST 6, the ID number is qualified for the access authorization only for "the hospital affairs management data", for example, and if the access authorization to "the drug library", for example, the database is not registered in "the hospital clerical staff information storage unit 21", the staff is rejected even though the staff requests for access to "the drug library" through one's ID number.

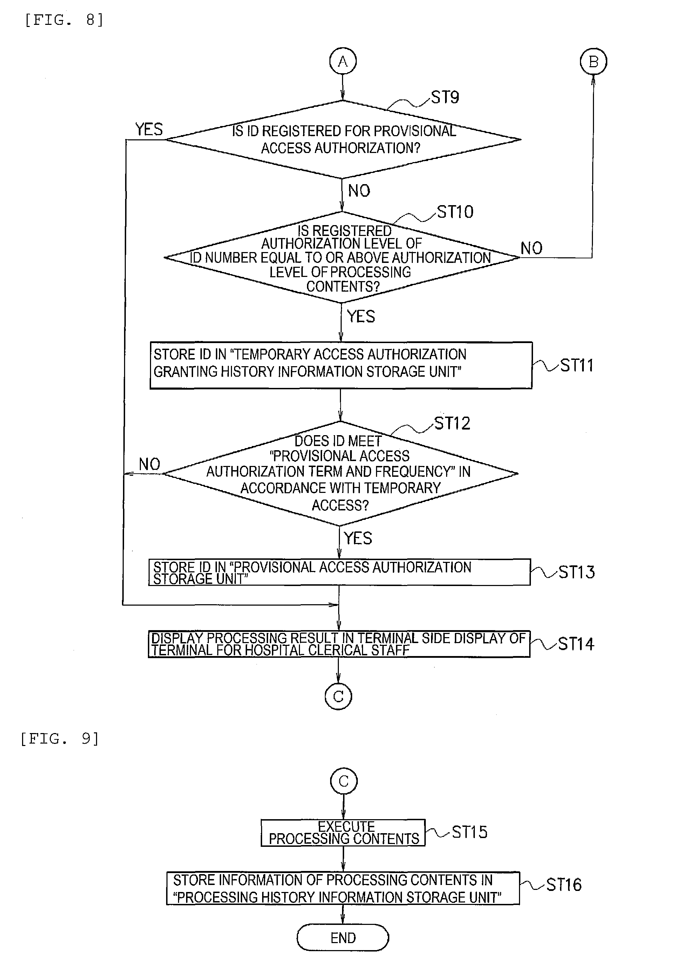

Subsequently, the procedure proceeds to ST 9 in Fig. 8, and "provisional access authorization" in ST 9 will be described later. In ST 9, if the ID number is not qualified for "the provisional access authorization", the procedure proceeds to ST 10.

In ST 10, the registered authorization level of the ID number, that is, information such as "the authorization level 6" or the like, for example, "the authorization level" stored in "the hospital clerical staff information storage unit 21" is acquired, thereby being compared to "the authorization level 3" of "composing/editing of the drug library" which is the processing contents. Then, it is judged whether or not the registered authorization level of the ID number is equal to or above "the authorization level 3" of "composing/editing of the drug library".

Specifically, "the access authorization judgment processing unit (program) 32" performs the judgment thereof.

In ST 10, when it is judged that the registered authorization level of the ID number is not equal to or above "the authorization level 3" of "composing/editing of the drug library", it is judged that there is no need to exceptionally approve the access authorization for the database which is not previously granted in advance, thereby denying a request for access to the database, that is, "composing/editing" of "the drug library" in the above example.

In this case, in order for hospital clerical staff of the ID number to have access to "the drug library" and to execute processing of "composing/editing", the hospital clerical staff needs to have a request to an administrator or the like so as to change stored contents in "the hospital clerical staff information storage unit 21" in Fig. 4. Accordingly, less needed exceptional access or the like of hospital clerical staff can be limited. Thus, it is possible to ensure security in management of the medical information.

Meanwhile, in ST 10, when it is judged that the registered authorization level of the ID number is equal to or above "the authorization level 3" of "composing/editing of the drug library", it is considered that there is a need to approve exceptional access of the hospital clerical staff, and the procedure proceeds to ST 11.

In ST 11, the ID number is stored in "a temporary access authorization granting history information storage unit 24" in Fig. 4. In other words, accordingly, at least, one-time processing authorization, for example, temporal limitation is granted.

Then, accordingly, even though the access authorization of "the drug library" which is the database is not previously registered in "the hospital clerical staff information storage unit 21", the access is exceptionally approved. Thus, it is possible to prevent duties in the conduction from being hindered.

The medical information management system is configured that when exceptionally approving access as described above, access is approved only if the registered authorization level of the ID number is equivalent to or above a numerical value, that is, the rating degree information of the authorization level of the processing contents. Therefore, the medical information management system is configured that security in management of the medical information is ensured, and convenience of a user is improved.

Note that, "the temporary access authorization granting history information storage unit 24" is an example of "an access history information storage unit".

Subsequently, the procedure proceeds to ST 12. In ST 12, in accordance with the temporary access authorization granting history in ST 11, it is judged whether or not a condition for "a provisional access authorization term-frequency information storage unit 25" in Fig. 4 is fulfilled.

"The provisional access authorization" is registered information for approving access to the processing contents (for example, "composing/editing of the drug library" or the like) provisionally omitting the step of ST 10 (comparison of the authorization levels or the like), in a case where the access authorization is not previously registered in "the hospital clerical staff information storage unit 21" with respect to the input database requested for access, if "the temporary access authorization granting" in ST 11 is approved in a certain frequency (for example, equal to or more than five times) within a certain term (for example, seven days).

Therefore, if the ID number is approved by "the provisional access authorization", it is possible to have access to the target processing contents more promptly, thereby further improving convenience of a user.

Specifically, "a provisional access authorization registration judgment processing unit (program) 33" in Fig. 5 judges whether or not the ID number meets conditional information with reference to frequency information stored in "the temporary access authorization granting history information storage unit 24" and conditional information (for example, equal to or more than five times in seven days) stored in "the provisional access authorization term-frequency information storage unit 25".

In ST 12, if it is judged that the ID number meets the conditional information (for example, equal to or more than five times in seven days), the procedure proceeds to ST 13. In ST 13, the ID number is stored in "a provisional access authorization storage unit 26" together with the processing contents (for example, "composing/editing of the drug library").

Specifically, the operation is executed by "a provisional access authorization granting processing unit (program) 34" in Fig. 5.

In this manner, as the ID number is registered in "the provisional access authorization storage unit 26", there is no need to pass through ST 9 and ST 10 or the like in Fig. 8. Thus, processing can be smoothly performed.

Note that, judgment in ST 9 is executed by "a provisional access authorization confirmation processing unit (program) 35" in Fig. 5.

Therefore, "the provisional access authorization storage unit 26" is an example of "an exceptional access information storage unit".

Subsequently, the procedure proceeds to ST 14. In ST 14, a processing result (for example, "Has stored in the provisional access authorization storage unit") is displayed in "the terminal side display 53" of the terminal 1A for hospital clerical staff in Fig. 1, thereby notifying hospital clerical staff of the processing result.

Subsequently, the procedure proceeds to ST 15 in Fig. 9. In ST 15, the hospital clerical staff who has input the corresponding ID number is considered to be authorized to execute "composing/editing of the drug library", for example, the processing contents, thereby executing "composing/editing of the drug library" which is the processing contents.

Subsequently, the procedure proceeds to ST 16. In ST 16, "a processing history information storage unit 27" in Fig. 4 stores that the ID number executed "composing/editing of the drug library" which is the processing contents at a specific time (year, month, date, time, or the like).

Specifically, the operation is executed by "a processing history information input unit (program) 36" in Fig. 5.

As described above, in the present embodiment, even though the specific processing contents (composing/editing or the like) of the database such as "the drug library" or the like is not previously registered in "the hospital clerical staff information storage unit 21" to be accessible being associated with the ID number, execution thereof is approved on condition that certain authorization is granted. Therefore, it is possible to ensure security in management of the medical information and to improve convenience of a user.

In the present embodiment, as described above, by granting "the provisional access authorization" to the ID number, "composing/editing of the drug library" which is the processing contents can be promptly executed without comparing the authorization level in ST 10, convenience of a user is further improved.

However, meanwhile, unrestrictedly approved authorization after "the provisional access authorization" is granted once denotes the effect of an approval equivalent to registration in "the hospital clerical staff information storage unit 21" in Fig. 4. Thus, it is not preferable in the viewpoint of security in management of the medical information.

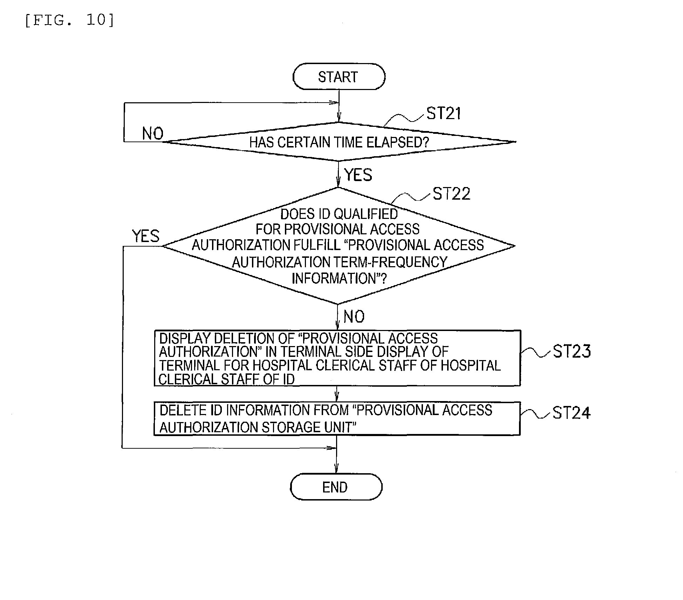

Therefore, in the present embodiment, as shown in Fig. 10, there is provided "a deleting step of registration of the provisional access authorization".

Firstly, in ST 21 in Fig. 10, "a provisional access authorization retention judgment processing unit (program) 41" in Fig. 6 operates, thereby determining whether or not a certain time has elapsed with reference to "the server side clocking device 1".

Then, when it is judged in ST 21 that a certain time has elapsed, the procedure proceeds to ST 22. In ST 22 as well, "the provisional access authorization retention judgment processing unit (program) 41" operates. Then, firstly, with reference to "the provisional access authorization storage unit 26" in Fig. 4, "the stored ID number" is extracted. Then, processing history information (processing frequency, time information, or the like) of the ID number is extracted from "the processing history information storage unit 27", thereby determining whether or not the processing history information fulfills the condition (equal to or more than five times in seven days, or the like) of "the provisional access authorization term-frequency information storage unit 25" in Fig. 4.

In ST 22, if it is judged that the condition (equal to or more than five times in seven days, or the like) of "the provisional access authorization term-frequency information storage unit 25" is not fulfilled, the procedure proceeds to ST 23.

In ST 23, deletion of "the provisional access authorization" is displayed in "the terminal side display 53" of the terminal 50A for hospital clerical staff of the hospital clerical staff of the ID number.

Subsequently, in ST 24, "a provisional access authorization deletion processing unit (program) 42" in Fig. 6 operates, thereby deleting the ID number from "the provisional access authorization storage unit 26".

In this manner, in the present embodiment, even after "the provisional access authorization" is registered once, the registration is limited within a range where a necessity is present. A use state thereof is monitored so as to delete "the unnecessary provisional access authorization", thereby securing security in management of the medical information.

However, the present invention is not limited to the embodiment described above. In the present embodiment, descriptions have been given exemplifying the infusion pump 2a or the like as a medical instrument, and the present invention is not limited thereto. It is possible to suitably apply to other medical instruments or the like such as a syringe pump or the like.

### Reference Signs List

1 ... medical information management system,
2a, 2b, 2c ... infusion pump,
10 ... hospital management server,
11 ... server control unit,
12 ... terminal side input device,
13 ... server side display,
14 ... server side communication device,
15 ... server side clocking device,
20 ... various-data item storage unit,
21 ... hospital clerical staff information storage unit,
22 ... input clerical staff ID information storage unit,
23 ... processing authorization level correlated information storage unit,
24 ... temporary access authorization granting history information storage unit,
25 ... provisional access authorization term-frequency information storage unit,
26 ... provisional access authorization storage unit,
27 ... processing history information storage unit,
30 ... various-data item processing unit (program) (1),
31 ... input information judgment processing unit (program),
32 ... access authorization judgment processing unit (program),
33 ... provisional access authorization registration judgment processing unit (program),
34 ... provisional access authorization granting processing unit (program),
35 ... provisional access authorization confirmation processing unit (program),
36 ... processing history information input unit (program), 40 ... various-data item processing unit (program) (2),
41 ... provisional access authorization retention judgment processing unit (program),
42 ... provisional access authorization deletion processing unit (program),
50A, 50B, 50C ... terminal for hospital clerical staff,
51 ... terminal control unit,
52 ... terminal side input device,
53 ... terminal side display,
54 ... terminal side communication device 54, and
55 ... terminal side various-data item storage unit or the like 55

## Claims

1. A medical information management apparatus comprising:
user identification information for identifying a user who uses medical information;
a plurality of items of first medical information that are obtained by segmenting the medical information into a plurality of types;
a plurality of items of second medical information that are obtained by segmenting the first medical information further into a plurality of types;
a third medical information storage unit that stores third medical information for causing the second medical information to be associated with degree identification information which indicates a degree of the second medical information; and
a user access associated information storage unit that stores the user identification information and user access associated information in which at least the user identification information is registered by causing the accessible first medical information to be associated with the degree identification information correlated to the user identification information, wherein
when the first medical information including the second medical information requested for access based on the user identification information is judged to be inaccessible based on the user access associated information, the degree identification information of the second medical information is specified based on the third medical information, and the specified degree identification information and the degree identification information of the user access associated information are compared to each other so as to judge whether or not access to the second medical information is possible.

2. The medical information management apparatus according to Claim 1, wherein
the degree identification information of the second medical information includes rating degree information which is a common criterion for each item of the first medical information, and
when the rating degree information of the specified degree identification information is not rated over the rating degree information of the degree identification information of the user access associated information, access requested based on the user identification information is approved on condition of temporal limitation.

3. The medical information management apparatus according to Claim 2, further comprising:
an access history information storage unit that stores history information of access permission approved on condition of the temporal limitation, wherein
when the history information satisfies a prejudged requirement, the access is permitted without comparing the specified degree identification information and the degree identification information of the user access associated information.

4. The medical information management apparatus according to Claim 3, wherein
when the permitted access is stored in an exceptional access information storage unit as exceptional access information without comparing the specified degree identification information and the degree identification information of the user access associated information, if the history information satisfies a prejudged condition, the exceptional access information is cancelled.

5. A medical information management system including the medical information management apparatus according to any one of Claims 1 to 4, the system comprising:
a terminal device that is used by a user, wherein
the medical information management apparatus is communicably connected to the terminal device.

6. A method of controlling a medical information management apparatus comprising:
user identification information for identifying a user who uses medical information;
a plurality of items of first medical information that are obtained by segmenting the medical information into a plurality of types;
a plurality of items of second medical information that are obtained by segmenting the first medical information further into a plurality of types;
a third medical information storage unit that stores third medical information for causing the second medical information to be associated with degree identification information which indicates a degree of the second medical information; and
a user access associated information storage unit that stores the user identification information and user access associated information in which at least the user identification information is registered by causing the accessible first medical information to be associated with the degree identification information correlated to the user identification information, wherein
when the first medical information including the second medical information requested for access based on the user identification information is judged to be inaccessible based on the user access associated information, the degree identification information of the second medical information is specified based on the third medical information, and the specified degree identification information and the degree identification information of the user access associated information are compared to each other so as to judge whether or not access to the second medical information is possible.
